# EUROPEAN PATENT SPECIFICATION

(11) **EP 1 086 938 B1**
(45) Date of publication and mention of the grant of the patent: **22.10.2003**
(21) Application number: 00111244.0
(22) Date of filing: 25.05.2000
(51) Int. Cl.: C07B 33/00, C07B 41/00, C07C 51/305, C07C 59/08, C07H 7/033

(54) **Oxidation of alcohols mediated by nitroxyl derivatives**
Durch Nitroxyl Derivate vermittelte Oxidation von Alkoholen
Oxydation d'alcools induit par dérivés nitroxyliques

(30) Priority: 21.09.1999 EP 99118600
(43) Date of publication of application: 28.03.2001
(73) Proprietor: Degussa AG, 40474 Düsseldorf (DE)
(72) Inventor: Hölderich, Wolfgang, Prof., 67227 Frankenthal (DE); Kochkar, Hafedh, Dr., 69006 Lyon (FR); Vanheertum, Rudolf, Dr., 63796 Kahl (DE); Morawietz, Marcus, Dr., 63755 Alzenau (DE)

(56) References cited:
- NOOY DE A E J ET AL: "ON THE USE OF STABLE ORGANIC NITROXYL RADICALS FOR THE OXIDATION OFPRIMARY AND SECONDARY ALCOHOLS" SYNTHESIS,DE,GEORG THIEME VERLAG. STUTTGART, 1 October 1996 (1996-10-01), pages 1153-1174, XP002072173 ISSN: 0039-7881
- SEMMELHACK M F ET AL: "Oxidation of alcohols to aldehydes with oxygen and cupric ion, mediated by nitrosonium ion" JOURNAL OF THE AMERICAN CHEMICAL SOCIETY,AMERICAN CHEMICAL SOCIETY, WASHINGTON, DC,US, vol. 106, no. 11, 1984, pages 3374-3376, XP002114880 ISSN: 0002-7863
- B. HINZEN ET AL.: "Polymer supported perruthenate (PSP): Clean oxidation of primary alcohols to carbonyl compounds using oxygen as cooxidant" SYNTHESIS, 1998, pages 977-979, XP002156866

## Description

The present invention is directed to a method for the oxidation of alcohols by the aid of a nitroxyl radical in the presence of a supported metal catalyst and a co-oxidant.

The oxidation of alcohols is very common among organic chemical reactions and is well established in many processes in industry. Especially, this holds true for the oxidation of primary alcohols to aldehydes or carboxylic acids.

This oxidation can advantageously be conducted in a catalytic manner. Among known catalytic methods the oxidation of alcohols with TEMPO and a co-oxidant became a very attractive tool for organic synthesis (Semmelhack et al. J. Am. Chem. Soc., 105 (1983) 4492-4494). The authors used the stable organic nitroxyl radical 2,2,6,6-tetramethylpiperidine-1-oxyl (TEMPO) as a mediator. The actual oxidant is the nitrosonium ion, which can be synthesised from TEMPO by several methods.

For example, soluable Fe(III)-ions oxidise MeO-TEMPO to immonium oxide, which shows good activity for the oxidation of benzyl alcohol in two phase system (aqueous phase/acetonitrile or methylene chloride). In this case, immonium oxide, which is a kind of ammonium salt, accelerates the reaction by acting as an anion-transport. The disadvantage of this method is that the Fe(III) was used in stoichiometric conditions, which is not feasible for industrial purpose in view of pollution and costs (Miyazawa, T. Endo., J. Molec. Catal., 31 (1985) 217-220).

Additionally, the nitroxyl radical can be oxidised by cupric salt to the oxo ammonium salt, which oxidises alcohols to the corresponding aldehydes, the hydroxylamine and a protonic acid. The hydroxylamine was in turn easily oxidised by cupric salts to give a nitroxyl radical. But it was necessary to trap the acid because it inhibits the catalytic oxidation system. Therefore, cupric hydroxide or pyridine were used as acid-trapping agents, again in stoichiometric amounts (M. F. Semmelhack, C. R. Schmid, D. A. Cortes, C. S. Chou., J. Am. Chem. Soc., 106 (1984) 3374-3376; T. Miyazawa, T. Endo., J. Molec. Catal., 32 (1985) 357-360).

Sulfonic acid, p-toluene sulfonic acid or 1(S)-(+)-camphor-10-sulfonic acid were used to generate the oxoammonium salt in methylene chloride as solvent. Thus, a large range of alcohols could be oxidised to the corresponding aldehydes or ketones. However, alcohols having a β-oxygen are not oxidised by oxo ammonium salts and those with a δ-oxygen react slowly. The drawback of this method is the use of an excess of nitroxyl radical and p-sulfonic acid (2 moles of nitroxyl radical per mole of alcohol was used) and results in many steps for the purification of the products (Z. H. Ma, J. M. Bobbitt, J. Org. Chem., 56 n°21 (1991) 6111-6114; J. A. Cella, J. A. Kelly, E. F. Kenehan., J. Org. Chem., 40 n°62 (1975) 1860). Again this process is disadvantage on industrial scale.

Primary alcohols are quantitatively oxidised to aldehydes under two phase conditions (methylene chloride-aqueous sodium hypochlorite) in the presence of catalytic amounts of 4-methoxy-2,2,6,6-tetramethylpiperidine-1-oxyl (MeO-TEMPO). Co-catalysis by bromide and buffering of pH at 8,6 with NaHCO₃ are also required (P. L. Anelli, C. Biffi, F. Montanari, S. Quici., J. Org. Chem., 52 (1987) 2259-2562; T. Inokuchi, S. Matsumoto, T. Nishigama, S. Torii., J. Org. Chem., 55 (1990) 462-466).

Many authors described a method for the oxidation of primary hydroxyl functions in an aqueous phase. The reaction is mediated by TEMPO and hypobromite is used as the regenerating oxidant, which in turn is regenerated by hypohalite as oxidant, preferably in the form of a salt, such as: lithium hypochlorite, sodium hypochlorite, potassium hypochlorite or calcium hypochlorite (A. E. J. de Nooy, A. C. Besemer, H. Van Bekkum., Recl. Trav. Chim. Pays-Bas., 113 (1994) 165-166; A. E. J. de Nooy, A. C. Besemer, Tetrahedron, 51 n° 29 (1995) 8023-8032; P. S. Chang, J. F. Robyt, J. Carbohydr. Chem., 15(7)(1996) 819-830).

More recently, the oxidation of primary hydroxyl groups of polysaccharide mediated by 2,2,6,6-tetramethylpiperidine-1-oxyl in water is disclosed in the specification of WO 95/07303 which relates to a process for producing completely carboxylated carbohydrates. In this process, carbohydrates having a carbonyl content of at least 75 % were oxidised using hypochlorite/bromide system. The amount of hypochlorite solution used was 2-2,4 moles per mole of monosaccharide unit. This clearly shows that the process produces a lot of halogenated salts, which cause some environmental pollution (AOX-waste).

The catalytic oxidation process of primary hydroxyl groups cited above has the following disadvantages:

### In two phase systems,

- (i) the oxidation of alcohol leads to the aldehyde only.
- (ii) the nitroxyl radical and the co-oxidant were used in stoichiometric amount.
- (iii) many steps for the purification of the products are required.

### In aqueous phase,

- (i) a large excess of co-oxidant was employed (2 moles and more of hypochlorite solution per mole of substrate).
- (ii) problems associated with this method are sooner or later, loss of the oxidizing power of the N-oxoammonium salts due to the concurrently generated hydrogen peroxide and molecular chlorine (T. Endo, T. Miyazawa, S. Shiihashi, M. Okawara, J. Am. Chem. Soc., 106 (1984) 3877-3878) and an accumulation of halogenated salts and AOX.

The present invention's task is to show another way to catalytically oxidise alcohols by the aid of an nitroxyl radical without showing the disadvantages involved in the oxidation procedures mentioned above.

This aim is reached by applying a process mentioned in claim 1. Further claims 2 to 18 are directed to preferable embodiments of the instant invention.

Through oxidation of alcohols by derivatives of the general formula (I) 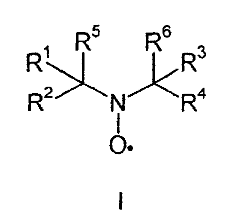 wherein R¹, R², R³, R⁴, R⁵, R⁶ are independent of each other (C₁-C₈)-alkyl, (C₁-C₈)-alkoxy, (C₆-C₁₈)-aryl, (C₇-C₁₉)-aralkyl, (C₆-C₁₈)-aryl- (C₁-C₈)-alkyl, (C₃-C₁₈)-heteroaryl, (C₄-C₁₉)-heteroaralkyl, (C₃-C₁₈)-heteroaryl-(C₁-C₈)-alkyl, (C₃-C₈)-cycloalkyl, (C₃-C₈)-cycloalkyl-(C₁-C₈)-alkyl, (C₁-C₈)-alkyl-(C₃-C₈)-cycloalkyl, or R⁵ and R⁶ are bonded together via a (C₁-C₃)-alkyl chain, which can be substituted by one or more R¹, N[(C₁-C₈)-alkyl]₂, (C₁-C₈)-amido, (C₁-C₈)-alkyloxy carboxyl, (C₆-C₁₈)-aryloxy carboxyl, nitrile, Hal, =O, part of a polymer, in the presence of a supported metal catalyst and a co-oxidant, one is able to gain oxidation products without having the drawbacks accompanied with the transformations known from the state of the art. Especially, the halogenated waste (AOX) and the hyperstoichiometric use of cost-effective and hardly disposable co-oxidants (Fe^{3+/2+}) can be omitted.

Preferably, a process is applied, wherein R¹ to R⁴ of formula (I) are (C₁-C₈)-alkyl and R⁵ and R⁶ are bonded via a (C₁-C₃)-alkyl chain, which can be substituted by one or more R¹, N[(C₁-C₈)-alkyl]₂, (C₁-C₈)-amido, (C₆-C₁₈)-aryloxy, (C₁-C₈)-alkyloxy carbonyl, (C₆-C₁₈)-aryloxy carbonyl, nitrile, Hal, =O, part of a polymer (e.g. T. Miyazawa, T. Endo., J. Polym. Scien., 23 (1985) 2487-2494).

Most preferably R¹ to R⁴ are methyl and R⁵ and R⁶ are bonded via a C₃-alkyl chain.

The amount of derivative of formula (I) applied is not critical. It should be used in an amount related to the cost-effectiveness of the process. Preferred is a process wherein the derivative of the general formula (I) is used within the range of 0.01 mol% to 10 mol%, more preferably between 0.1 mol% and 4 mol%, based on the substrate.

The process can be conducted with primary and secondary alcohols. According to their reactivity primary alcohols are oxidised much faster than secondary ones, which helps the skilled man to discriminate between both groups in a manner that primary alcoholic functions can be oxidised selectively in the presence of secondary alcoholic groups within the same molecule. However, the alcohol used in the process of the invention preferably comprises a primary alcoholic function.

Primary alcoholic residues are normally oxidised to the corresponding carboxylic acids or aldehyde depending on if water is used as reaction solvent or an organic medium (A. E. J. de Nony et al., Tetrahedron 1993, 51, 8023f). [What about synthesising aldehydes?]

In a preferred embodiment of the invention the supported metal catalyst comprises metals in an oxidation stage ±0 or metal oxides. Preferably, as elementary metals are used Silver, Chromium, Palladium , Bismuth, Iron, Vanadium, Copper, Cobalt, Nickel, Molybdenum, Manganese, Ruthenium or Osmium or alloys of the same. In addition, the metal oxides used can be MOₓ with M = Silver, Chromium, Palladium, Bismuth, Iron, Vanadium, Copper, Cobalt, Nickel, Molybdenum, Manganese, Ruthenium or Osmium or mixtures of the same or mixed metal oxides of the same.

The support itself is advantageously made out of micro or mesoporous materials. The micro porous material can be derived from zeolitic material and the mesoporous material can be silica, alumina, alumino phosphate, titanium oxide or celite or carbon. The zeolitic material used may be zeolites such as X, L, Y, mordenite, mesoporous materials as of the M41-S family (MCM-41, MCM-48, MCM-50) or silica, alumina, amorphous silica-alumina, aluminophosphate, titanium oxide, zirconium oxide and celite.

The ratio of metal to support lies within the range of 0.01 wt% to 60 wt%, preferably between 0.05 wt% to 50 wt% and more preferably between 0.1 wt% to 30 wt%.

The co-oxidant used in this invention has to be strong enough to oxidise the corresponding hydroxylamin of formula (I) to the nitrosonium ion in the presence of the catalyst.

On the other hand it has to be mild enough not to destroy the alcohol to be oxidised. All oxidants with an Eₒ of > 0,3 and less than 2,5 are feasible. Preferably, the co-oxidant is selected from a group consisting of hydrogen peroxide, oxone, ozone, perborate, percarbonate, oxygen, N-methyl morpholine-4-oxid, peroxodisulfate or caroate.

The ratio of co-oxidant to substrate is not critical but shall be kept as low as possible for economic and ecological reasons. Normally, it lies within the range of 1 to 20, preferably between 1-10, more preferably between 1-2, equivalents.

The process of the invention is preferably conducted within a solvent. More preferably it is carried out in water or an organic solvent, most preferably acetone, acetonitrile, benzene, dioxane, diglyme, tetrahydrofurane or water or mixtures thereof.

If water is part of the solvent the pH shall lie within the range of 2 to 12, preferably between 8.5 to 10.

The temperature of the reaction may be selected according to the molecules or reaction conditions applied and is not critical. Normally, it lies within the range of -30 °C to 100 °C, preferably between -10 °C to 50 °C.

The catalyst can be recovered after and can be reused for subsequent oxidation reactions.

In proposed mechanism the catalytic system works as exemplified in the following scheme:

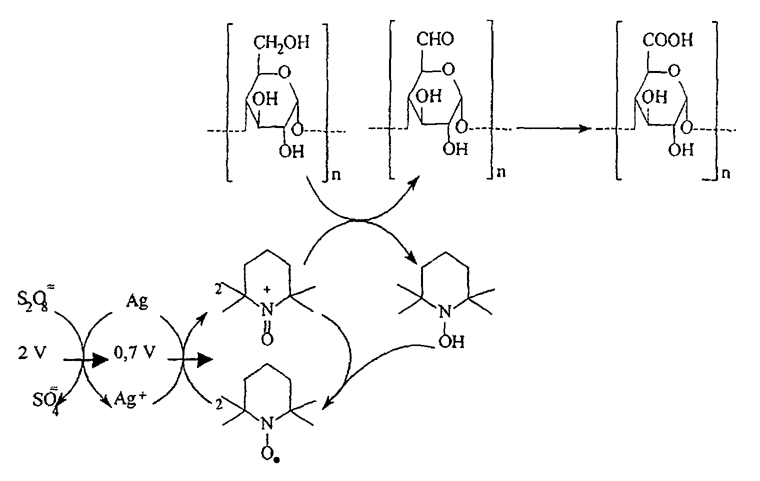

The catalyst reoxidises the hydroxyl amine, formed during the oxidation of the alcohol, to the corresponding nitroxyl radical or the corresponding nitrosonium ion, which subsequently is again able to oxidise an alcohol function. In order to close the cycle, it is important to use a primary co-oxidant to reoxidise the reduced metal (or its equivalent).

The method according to the invention can be used for oxidising primary alcohols of very large range. Polyols are readily oxidised at the primary hydroxyl groups in preference to the secondary and/or tertiary hydroxyl groups.

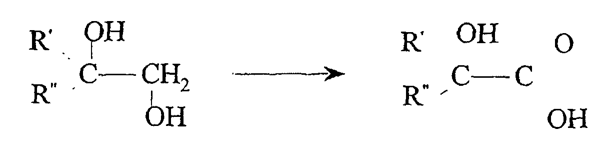

In particular, the method is suitable for the oxidation of water soluble polyols. The preferred substrate for the present oxidation is 1,2-propandiol (R' = Me and R" = H).

The method according to the present invention can advantageously be employed for the oxidation of primary alcohols. Preferred alcohols are such as: n-butanol, cyclohexanol, cyclopentanol, 3-methylcyclohexanol, 2-methylcyclohexanol, 1-hexanol, 1-butanol, octanol, dodecanol, octanol, (±)-2-norbornane-methanol, (1R)-(-)-nopol, piperonyl alcohol, cinnamyl alcohol, famesol, nerol, geraniol. The more preferred substrates for the present oxidation reaction are: 1-butanol and 1-hexanol.

The method according to the present invention can be used for the regioselective oxidation of a wide range of carbohydrates, if they have primary hydroxyl groups. Examples of carbohydrates include: methyl-α-D-glucopyranoside, methyl-β-D-glucopyranoside, octyl-β-D-glucopyranoside, sucrose, dodecyl-D-glucopyranoside, α-D-glucopyranoside-1-(disodium phosphate), α-α-trehalose. The preferred substrates for the present oxidation are: methyl-α-D-glucopyranoside and sucrose and, especially, methyl-α-D-glucopyranoside.

As compared with the conventional processes, the process of the present invention has the following merits:
- extremely high activity and selectivity are obtained in the oxidation of primary alcohol or primary hydroxyl groups e.g. in the case of carbohydrates. More particularly, primary hydroxyl groups of methyl-α-D-glucopyranoside are oxidised regioselectively in presence of secondary ones, side reactions are diminished,
- small amounts of nitroxide radical are used (0.1-4 mol% per mol of substrate),
- the hypochlorite/bromide system used before was replaced by a an environmentally benign catalytic system,
- the carboxylic acid salts obtained have a high purity, which requires no further purification.

There come into consideration as (C₁-C₈)-alkyl radicals linear or branched methyl, ethyl, n-propyl, isopropyl, n-butyl, isobutyl, sec.-butyl, tert.-butyl, pentyl, hexyl, heptyl or octyl, including all their possible isomers. The radical (C₁-C₈)-alkoxy corresponds to the radical (C₁-C₈)-alkyl, with the proviso that it is bonded to the ring *via* an oxygen atom.
(C₃-C₈)-cycloalkyl is to be understood as being cyclopropyl, cyclobutyl, cyclopentyl, cyclohexyl or cycloheptyl radicals.

Hal means fluorine, chlorine, bromine and iodine.

(C₆-C₁₈)-Aryl denotes arylic species with 6 to 18-C-atoms, like phenyl, naphthyl, phenanthryl.
(C₇-C₁₉)-aralkyl are arylic radicals connected via a (C₁-C₈)-alkyl radikal with the molecule of respect.
(C₃-C₁₈)-heteroaryl are arylic molecules in which at least one C-atom is substituted by a heteroatom like N, O, P, S. Molecules, which come into consideration, are pyrolyl, furyl, pyridyl, imidazolyl, chinolinyl, etc.
(C₄-C₁₉)-heteroaralkyl are heteroarylic species bonded via a (C₁-C₈)-alkyl radical with the molecule of respect.
(C₁-C₃)-alkyl chain represents groups like methenyl, 1,2-ethenyl, 1,3-propenyl.
(C₁-C₈)-alkoxy carboxyl is a (C₁-C₈)-alkoxy-group bonded via a carboxyl function to the molecule of respect.
(C₆-C₁₈)-aryloxy carboxyl is a (C₆-C₁₈)-aryl-group bonded via a carboxyl function to the molecule of respect (C₁-C₈)-amido represents a (C₁-C₈)-alkyl radical, which is linked to the molecule in question via a amido group.

### Examples :

The following examples will further illustrate the present invention.

### General description :

Experiments were performed in a thermostated glass batch reactor of 250 ml volume, equipped with a condenser, a thermometer and a magnetic stirrer. The pH was kept constant using a pH-meter (titroline alpha) coupled to a pH control unit and an automatic burette (Metrohm 655, 20 ml piston) containing 1M KOH.

### Oxidation procedure :

A defined amount of substrate (5 mmol) was dissolved in 100 ml of water. Then 30 mg (0,19 mmol) of TEMPO and 10 mmol of co-oxidant were added. The system was equilibrated at 298 K and pH 9.5. The catalyst was instantaneously added to the mixture. The pH of the solution during the reaction was kept constant at 9.5 by automatic titration with 1M KOH solution. At the end of the reaction, the pH-value was adjusted at pH = 8 by adding hydrochloric acid to pH 8. Then the catalyst was recovered by filtration, water and TEMPO were removed under vacuum at 313 K. The product mixture was dried overnight under vacuum at room temperature. The oxidation degree of the substrate was determined by gas chromatography after silylation.

For silylation, e.g. 10 mg of the oxidised sugar was introduced in a gas chromatography tube and 600 mL of pyridine was added. The solution was stirred until the mixture was totally solubilised. Then 600 mL of hexamethyldisilazane and 150 mL of trifluoroacetic acid were introduced. The mixture was placed in an oven at 363 K for 30 min. Then n-decane was added as the internal standard for quantitative analysis. The products were analyzed by GC SIEMENS equipped with capillary column ultra chromapack 25 m length and a flame ionization detector (FID).

### Preparation of the catalysts:

### - Metal-supported on Al₂O₃, AlPO₄ and NaY

Ag-Na-Y, Ag-AlPO₄ and Ag-Al₂O₃ were prepared by incipient wetness impregnation. To a solution containing the support (10 g), silver nitrate was added. The mixture was stirred during 15 h and then filtrated, washed and dried overnight at 373 K. Then, the catalyst was calcined under air at 773 K during 6 h. The composition of the catalysts was determined by Inductive Coupled Plasma (ICP) after dissolution of the solids. The specific BET surface and average pore diameter were obtained from the adsorption isotherms of nitrogen at 77 K, using micromeritrics automatic volumeter. The samples were first evacuated at 473 K for 16h. The results are reported in Table 1.

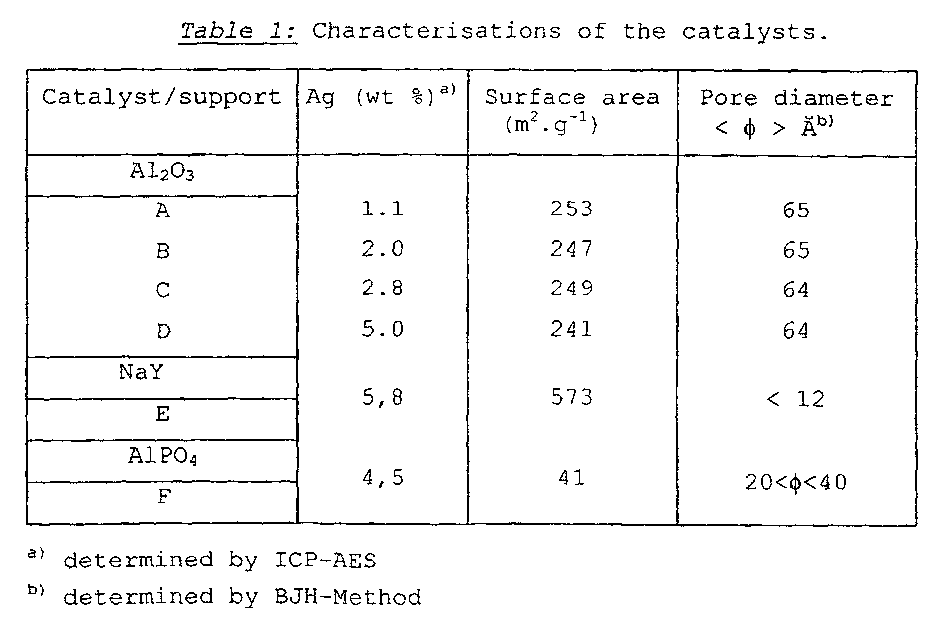

### - Preparation of the silver carbonate on celite

The supporting material celite is purified by washing with methanol containing 10 % concentrated hydrochloric acid, and then with distilled water until the mother liquor was neutral. Then the material was dried at 393 K.

To a mechanically stirred solution of silver nitrate (1,85 mmol, 0,31 g) in distilled water (100 ml) purified celite (10 g) is added. To the stirred suspension, a solution of potassium hydrogen carbonate (0,15 g) in distilled water (100 ml) is slowly added. After complete addition, stirring is continued for a further 10 min. A yellow-green precipitate formed is collected by filtration and dried under reduced pressure on a rotary evaporator for several hours. The catalyst contains 0,17 mmol of silver per gram of celite (0,088 mmol of Ag₂CO₃ per gram of reagent). The same procedure was repeated for another percentage of silver (See Table 2).

**Table 2**

| | Catalyst G | Catalyst H |
|---|---|---|
| AgNO₃ (g) | 0,31 | 11,34 |
| NaHCO₃ (g) | 0,15 | 5,6 |
| mmol Ag₂CO₃/ g celite | 0,088 | 1 |
| Ag^{a)} (wt.%) | 2 | 32 |

| | | |
|---|---|---|
| ^{a)}: determined by ICP-AES | | |

### - Others catalysts:

M-Na-Y or M-Al₂O₃ catalysts with M = Cu, Co, V, Mn, Ni, Fe, Bi, Pd, and/or Ru were prepared by impregnation at room temperature. Bimetallic catalysts Ag-Cu and Ag-Co were also prepared. The composition of the catalysts was determined by Inductive Coupled Plasma (ICP) after dissolution of the solids. The results are reported in Table 3.

**Table 3**

| Catalysts | Name | Metal (wt.%) | Surface area (m².g⁻¹) |
|---|---|---|---|
| Ag₂O | I | | - |
| Cu-Y | J | 1.5 | 495 |
| Co-Y | K | 1.5 | 620 |
| V-Y | L | 3.0 | 293 |
| Mn-Y | M | 1.5 | 460 |
| Cu-Al₂O₃ | N | 5.0 | 203 |
| Ni-Al₂O₃ | O | 3.8 | 228 |
| Mn-Al₂O₃ | P | 3.3 | 218 |
| Co-Al₂O₃ | R | 3.2 | 209 |
| Co-γ-Al₂O₃*⁾ | S | 4.2 | - |
| α-Fe/Bi-Al₂O₃ | T | 0.9 and 5.3 | 126 |
| Pd/Ag-Al₂O₃ | U | - | |
| Ru-Al₂O₃ | V | 1.2 | 243 |
| [AgCu]O | W | 54 | - |
| [AgCo]O | X | 59 | - |

| | | | |
|---|---|---|---|
| *⁾obtained by calcination of catalyst R at 1073 K under air. | | | |

### - Physico-chemical characterisation

The composition of the catalysts was determined by Inductive Coupled Plasma (I.C.P) after dissolution of the solids. The specific BET surface and average pore diameter were obtained from the adsorption isotherms of nitrogen at 77 K, using micromeritrics automatic volumeter. The samples were first evacuated at 473 K for 16 h. X-Ray diffraction study was performed on SIEMENS 5000 instrument equipped with Cu lamp (λ _{cu} = 1.54 Å). The spectra were recorded from 2θ = 5° to 2θ = 100°.

The amount of acidic sites was determined by programmed temperature desorption TPD of ammonia. On samples previously calcined at 673 K under Helium flow (over night). The ammonia was adsorbed during 15 min at 373 K. The temperature was increased using a ramp of 10 °C/mins to 1073 K. The desorbed ammonia was titrated by HCl (0.025 M) using a special titrometer coupled to a pH control unit and an automatic burette containing the acid. During the desorption, temperature, pH and HCl consumption were recorded.

### Example 1:

Methyl-α-D-glucopyranoside (0,97 g, 5 mmol) was dissolved in 100 ml water. Then 30 mg (0,19 mmol) of TEMPO and 2,28 g (10 mmol) of ammonium peroxodisulfate were added. The system was equilibrated at 298 K and pH 9,5 under vigorous stirring (1000 rpm). Then 500 mg of catalyst A was added to the mixture (time zero). The pH of the reaction was kept constant at 9,5 by automatic titration with 1M KOH solution.

For comparison, catalyst A and ammonium peroxodisulfate were replaced by catalyst I (10 mmol, 2,31 g). At the end of the reaction, the pH-value was adjusted to 8 by adding hydrochloric acid.

Then the catalyst was recovered by filtration, water and TEMPO were removed under vacuum at 313 K. The product mixture was dried overnight under vacuum at room temperature. The oxidation degree was determined by gas chromatography after silylation. The results are reported in table 4.

**Table 4**

| Oxidation of methyl-α-D-glucopyranoside over silver catalysts. | | |
|---|---|---|
| Catalyst | Conversion (%) | Selectivity (%.mol) |
| catalyst I (0.5) | 49 | 95 |
| catalyst A (108) | 70 | 97 |
| pH= 9,5, 298 K, α-MDG/TEMPO= 26 (mol), | | |
| ( ):is the molar ratio between the substrate and silver | | |

The selectivity of the catalyst for the oxidation of methyl-α-D-glucopyranoside to methyl-α-D-glucopyranosiduronic acid is very good. The oxidation was found to be stoichiometric, when catalyst I is used in absence of peroxodisulfate. The silver oxide was reduced to metallic silver.

Using catalyst A in combination with peroxodisulfate, with a molar ratio α-MDG to Ag of 108, a very high selectivity to methyl-α-D-glucopyrasiduronic acid 97 % is obtained at a conversion of 70 %. In this new procedure the silver works as a catalyst.

### Example 2:

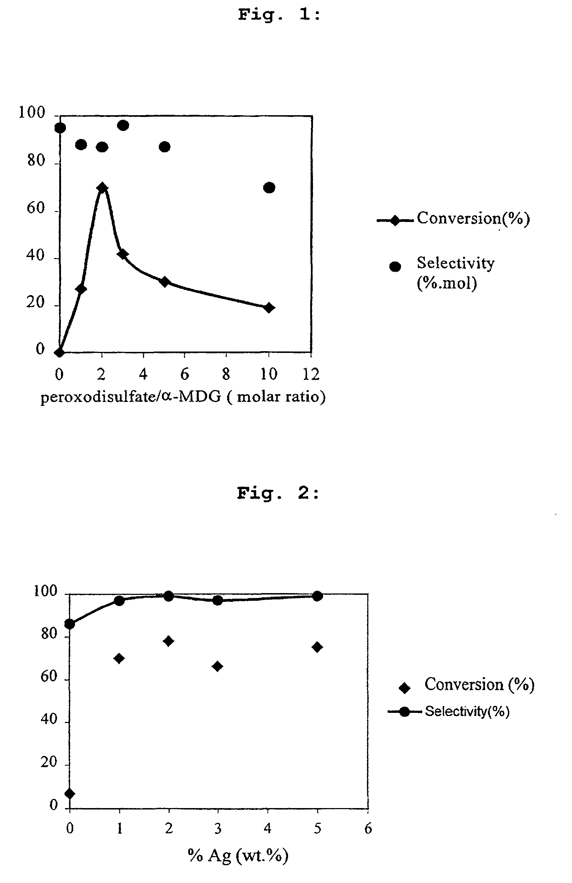

Example 1 was repeated, except that the ratio of peroxodisulfate to α-MDG was varied. The effect of the amount of peroxodisulfate on the catalytic properties of Ag-Al₂O₃ is shown in Figure 1. With a molar ratio of peroxodisulfate:α-MDG close to 2, a selectivity of 99 % at a conversion of approximately 80 mol.% is obtained.

### Example 3:

Example 1 was repeated except that the percentage of silver was varied: 1, 2, 3, and 5 wt%, respectively. Applying a molar ratio of α-MDG:peroxodisulfate:TEMPO of 1:2:0,1. The result is shown in Figure 2.

α-MDG oxidation is very low in absence of silver around 7% conversion. Whereas, the conversion increases with silver percentages and reaches a plateau at 78 % conversion with a selectivity of 99 % to methyl-α-D-glucopyrasiduronic acid.

### Example 4:

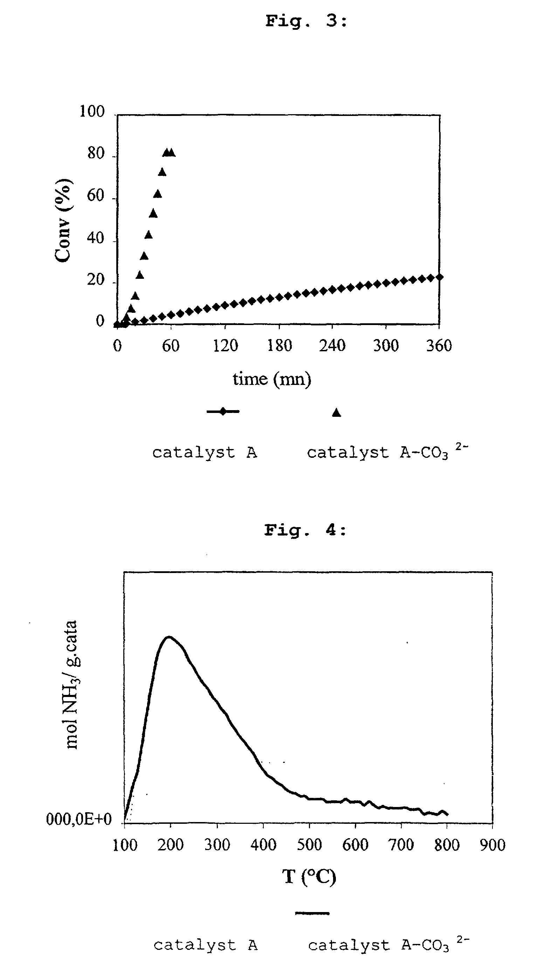

Example 1 was repeated, except that the catalyst A was treated with carbonate, using a molar ratio of carbonate-silver of 0,5. In Figure 3, the conversion as a function of reaction time is demonstrated for the parent catalyst A and for catalyst A-CO₃. The initial rate increases by a factor of 10 in the case of catalyst A-CO₃ .This result can be interpreted by the fact that in presence of carbonate, the charge density on silver decreases and more Lewis acid sites are created. This hypothesis was verified by TPD of ammonia (Figure 4).

### Example 5:

α-MDG was oxidized in the presence of 500 mg of catalyst E (containing 5.8 wt% of silver) in the same manner as in example 1. The α-MDG conversion was 74 % with a selectivity of 99 mol.% to methyl-α-D-glucopyrasiduronic acid.

### Example 6:

α-MDG was oxidized in the presence of 500 mg of freshly prepared catalysts G and H containing different amounts of silver carbonate. The reaction was carried out in the same manner as described in example 1. The results are shown in Table 5.

**Table 5**

| | Catalyst G | Catalyst H |
|---|---|---|
| mmol Ag₂CO₃/ g celite | 0,088 | 1 |
| Conversion (%) | 51 | 90 |
| Selectivity (mol.%) | 99 | 99 |

### Example 7:

Various supported metal catalysts were used for the oxidation of methyl-α-D-glucopyranoside using the following conditions: methyl-α-D-glucopyranoside (0,97 g, 5 mmol) was dissolved in 100 ml water. Then 30 mg of TEMPO and (2,28 g, 10 mmol) of ammonium peroxodisulfate were added. The system was equilibrated at 298 K and pH 9,5 under vigorous stirring (1000 rpm). Then 500 mg of supported metal catalysts and/or mixed oxides were added to the mixture (time zero). The pH of the reaction was kept constant at 9,5 by automatic titration with 1M KOH solution. At the end of the reaction, the mixture was neutralised by adding hydrochloric acid to a pH 8. Then the catalyst was recovered by filtration, water and TEMPO were removed under vacuum at 313 K. The product mixture was dried overnight under vacuum at room temperature. The oxidation degree was determined by gas chromatography after silylation. The results are shown in Table 6.

**Table 6**

| Catalysts | Conversion (%) | Selectivity (mol.%) |
|---|---|---|
| B | 78 | 99 |
| E | 74 | 99 |
| F | 24 | 86 |
| J | 28 | 99 |
| K | 60 | 99 |
| L | 15 | 99 |
| M | 10 | 99 |
| N | 24 | 99 |
| O | O | - |
| P | 19 | 99 |
| R | 21.5 | 99 |
| S | 12 | 97 |
| T | 18.7 | 97 |
| U | 62 | 80 |
| V | 5 | 97 |
| W | 60 | 95 |
| X | 65 | 96 |

### Example 8:

The catalyst W with 54wt% silver was used in two successive oxidation runs with a fresh charge of methyl-α-D-glucopyranoside, peroxodisulfate and TEMPO solution. The reaction was carried out in the same conditions as described in the example 7. The results are shown in Table 7.

**Table 7**

| Number of repetition | Conversion (%) | Selectivity (mol.%) |
|---|---|---|
| 1 | 60 | 95 |
| 2 | 60 | 95 |

### Example 9:

1,2-propandiol (0,38 g, 5 mmol) was dissolved in 100 ml water. Then 30 mg of TEMPO and (2,28 g, 10 mmol) of ammonium peroxodisulfate were added. The system was equilibrated at 298 K and pH 9,5 under vigorous stirring (1000 rpm). Then 500 mg of catalyst D was added to the mixture (time zero). The pH of the reaction was kept constant at 9,5 by automatic titration with 1M KOH solution.

At the end of the reaction, the pH-value was adjusted to 8 by adding hydrochloric acid. Then the catalyst was recovered by filtration, water and TEMPO were removed under vacuum at 313 K. The product mixture was dried overnight under vacuum at room temperature. The oxidation degree was determined by gas chromatography after silylation.

At the end of the reaction, the selectivity of the lactic acid is 75 mol.% at 90 % conversion of 1,2-propandiol.

### Example 10 :

Example 9 was repeated except that the catalyst D was replaced by Ag-carbonate on celite, catalyst H, containing 1mmol Ag₂CO₃/g celite. At the end of the reaction, the selectivity of the lactic acid is 60 mol.% at 75 % conversion of the 1,2-propandiol.

### Example 11 :

Example 10 was repeated except that the ammonium peroxodisulfate was replaced by oxone (10 mmol, 3g). At the end of the reaction, the selectivity of the lactic acid is 99 mol.% at 8 % conversion of the 1,2-propandiol.

### Example 12 :

Example 9 was repeated except that the catalyst D was replaced by catalyst E containing 5.8 wt % silver. At the end of the reaction, the selectivity of the lactic acid is 64 mol.% at 60 % conversion of the 1,2-propandiol.

### Example 13:

Example 12 was repeated except that the ammonium peroxodisulfate was replaced by oxone (10 mmol, 3g). At the end of the reaction, the selectivity of the lactic acid is 55 mol.% at 18 % conversion of the 1,2-propandiol.

## Claims

1. Process for the oxidation of alcohols by derivatives of the general formula (I) 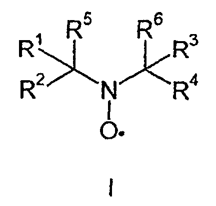 wherein R¹, R², R³, R⁴, R⁵, R⁶ are independent of each other (C₁-C₈)-alkyl, (C₁-C₈)-alkoxy, (C₆-C₁₈)-aryl, (C₇-C₁₉)-aralkyl, (C₆-C₁₈)-aryl-(C₁-C₈) -alkyl, (C₃-C₁₈)-heteroaryl, (C₄-C₁₉)-heteroaralkyl, (C₃-C₁₈)-heteroaryl-(C₁-C₈)-alkyl, (C₃-C₈)-cycloalkyl, (C₃-C₈)-cycloalkyl-(C₁-C₈)-alkyl, (C₁-C₈)-alkyl-(C₃-C₈)-cycloalkyl, or R⁵ and R⁶ are bonded together via a (C₁-C₃)-alkyl chain, which can be substituted by one or more R¹, N[(C₁-C₈)-alkyl]₂, (C₁-C₈)-amido, (C₁-C₈)-alkyloxy carboxyl, (C₆-C₁₈)-aryloxy carboxyl, nitrile, Hal, =O, part of a polymer,
in the presence of a supported metal catalyst and a co-oxidant.

2. Process according to claim 1,
wherein R¹ to R⁴ are (C₁-C₈)-alkyl and R⁵ and R⁶ are bonded via a (C₁-C₃)-alkyl chain, which can be substituted by one or more R¹, N[(C₁-C₈)-alkyl]₂, (C₁-C₈)-amido, (C₁-C₈)-alkyloxy carboxyl, (C₆-C₁₈)-aryloxy carboxyl, nitrile, Hal, =O, part of a polymer.

3. Process according to claim 1 and/or 2,
wherein R¹ to R⁴ are methyl, R⁵ and R⁶ are bonded via a C₃-alkyl chain.

4. Process according to one or more of claims 1 to 3,
wherein the derivative of the general formula (I) is used within the range of 0.01 mol% to 10 mol%, preferably between 0.1 mol% and 4 mol%, based on the substrate

5. Process according to one or more of claims 1 to 4,
wherein the alcohol used comprises a primary alcoholic function.

6. Process according to claim 5,
wherein the alcohol is oxidised to the corresponding carboxylic acid.

7. Process according to claim 1,
wherein the supported metal catalyst comprises metals in an oxidation stage ±0 or metal oxides.

8. Process according to claim 7,
wherein the metals used are Silver, Chromium, Palladium , Bismuth, Iron, Vanadium, Copper, Cobalt, Nickel, Molybdenum, Manganese, Ruthenium or Osmium or alloys of the same.

9. Process according to claim 7,
wherein the metal oxides used are MOₓ with M = Silver, Chromium, Palladium, Bismuth , Iron, Vanadium, Copper, Cobalt, Nickel, Molybdenum, Manganese, Ruthenium or Osmium or mixtures of the same or mixed metal oxides of the same.

10. Process according to one or more of claims 1 to 9,
wherein the support used is micro or mesoporous materials.

11. Process according to claim 10,
wherein the micro porous material can be derived from zeolitic material and the mesoporous material can be silica, alumina, amorphous silica-alumina, alumino phosphate, titanium oxide or celite or carbon.

12. Process according to one or more of claims 1 to 11,
wherein the ratio of metal to support lies within the range of 0.05 wt% to 50 wt%.

13. Process according to one or more of claims 1 to 12,
wherein the co-oxidant is selected from the group consisting of hydrogen peroxide, oxone, ozone, perborate, percarbonate, oxygen, N-methyl morpholine-4-oxid, peroxodisulfate or caroate.

14. Process according to one or more of claims 1 to 13,
wherein the ratio of co-oxidant to substrate lies within the range of 1 to 20, preferably between 1-10, equivalents.

15. Process according to one or more of claims 1 to 14,
wherein the reaction is carried out in water or an organic solvent, preferably acetone, acetonitrile, benzene, dioxane, diglyme, tetrahydrofurane or mixtures thereof.

16. Process according to claim 15,
wherein if water is part of the solvent the pH lies within the range of 2 to 12, preferably between 8.5 to 10.

17. Process according to one or more of claims 1 to 16,
wherein the temperature of the reaction lies within the range of -30 °C to 100 °C, preferably between -10 °C to 50 °C.

18. Process according to one or more of the preceding claims,
wherein the catalyst is recovered after and is reused for subsequent oxidations.

## Patentansprüche

1. Verfahren zur Oxidation von Alkoholen durch Derivate der allgemeinen Formel (I) 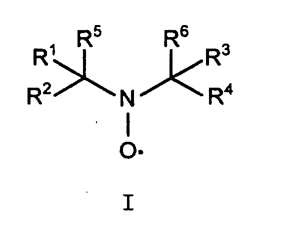 wobei R¹, R², R³, R⁴, R⁵, R⁶ unabhängig voneinander (C₁-C₈)-Alkyl, (C₁-C₈)-Alkoxy, (C₆-C₁₈)-Aryl, (C₇-C₁₉)-Aralkyl, (C₆-C₁₈)-Aryl- (C₁-C₈)-Alkyl, (C₃-C₁₈)-Heteroaryl, (C₄-C₁₉)-Heteroaralkyl, (C₃-C₁₈)-Heteroaryl-(C₁-C₈)-Alkyl, (C₃-C₈)-Cycloalkyl, (C₃-C₈)-Cycloalkyl-(C₁-C₈)-Alkyl, (C₁-C₈)-Alkyl-(C₃-C₈)-Cycloalkyl darstellen oder R⁵ und R⁶ über eine (C₁-C₃)-Alkylkette, die durch ein oder mehrere R¹, N[(C₁-C₈)-Alkyl]₂, (C₁-C₈)-Amido, (C₁-C₈)-Alkyloxycarboxyl, (C₆-C₁₈)-Aryloxycarboxyl, Nitril, Hal, =O, Teil eines Polymers substituiert sein kann, verbunden sind, in Gegenwart eines Metall-Trägerkatalysators und eines Cooxidans.

2. Verfahren nach Anspruch 1, wobei R¹ bis R⁴ (C₁-C₈)-Alkyl darstellen und R⁵ und R⁶ über eine (C₁-C₃)-Alkylkette, die durch ein oder mehrere R¹, N[(C₁-C₈)-Alkyl]₂, (C₁-C₈)-Amido, (C₁-C₈)-Alkoxycarboxyl, (C₆-C₁₈)-Aryloxycarboxyl, Nitril, Hal, =O, Teil eines Polymers substituiert sein kann, gebunden sind.

3. Verfahren nach Anspruch 1 und/oder 2, wobei R¹ bis R⁴ Methyl darstellen, R⁵ und R⁶ über eine C₃-Alkylkette gebunden sind.

4. Verfahren nach einem oder mehreren der Ansprüche 1 bis 3, wobei das Derivat der allgemeinen Formel (I) im Bereich von 0,01 Mol-% bis 10 Mol-%, bevorzugt zwischen 0,1 Mol-% und 4 Mol-% bezogen auf das Substrat verwendet wird.

5. Verfahren nach einem oder mehreren der Ansprüche 1 bis 4, wobei der verwendete Alkohol eine primäre Alkoholfunktion umfasst.

6. Verfahren nach Anspruch 5, wobei der Alkohol zur entsprechenden Carbonsäure oxidiert ist.

7. Verfahren nach Anspruch 1, wobei der Metall-Trägerkatalysator Metalle in einer Oxidationsstufe ± 0 oder Metalloxide umfasst.

8. Verfahren nach Anspruch 7, wobei die verwendeten Metalle Silber, Chrom, Palladium, Bismut, Eisen, Vanadium, Kupfer, Kobalt, Nickel, Molybdän, Mangan, Ruthenium oder Osmium oder Legierungen derselben sind.

9. Verfahren nach Anspruch 7, wobei die verwendeten Metalloxide MOₓ mit M = Silber, Chrom, Palladium, Bismut, Eisen, Vanadium, Kupfer, Kobalt, Nickel, Molybdän, Mangan, Ruthenium oder Osmium oder Gemische derselben oder gemischte Metalloxide derselben sind.

10. Verfahren nach einem oder mehreren der Ansprüche 1 bis 9, wobei der verwendete Träger aus mikro- oder mesoporösen Materialien besteht.

11. Verfahren nach Anspruch 10, wobei das mikroporöse Material von Zeolithmaterial hergeleitet werden kann und das mesoporöse Material Siliciumdioxid, Aluminiumoxid, amorphes Silica-Alumina, Aluminophosphat, Titanoxid oder Celit oder Kohlenstoff sein kann.

12. Verfahren nach einem oder mehreren der Ansprüche 1 bis 11, wobei das Verhältnis von Metall zu Träger im Bereich von 0,05 Gew.-% bis 50 Gew.-% liegt.

13. Verfahren nach einem oder mehreren der Ansprüche 1 bis 12, wobei das Cooxidans aus der Gruppe ausgewählt ist, bestehend aus Wasserstoffperoxid, Oxon, Ozon, Perborat, Percarbonat, Sauerstoff, N-Methylmorpholin-4-oxid, Peroxodisulfat oder Caroat.

14. Verfahren nach einem oder mehreren der Ansprüche 1 bis 13, wobei das Verhältnis von Cooxidans zu Substrat im Bereich von 1 bis 20, bevorzugt zwischen 1 und 10 Äquivalenten liegt.

15. Verfahren nach einem oder mehreren der Ansprüche 1 bis 14, wobei die Reaktion in Wasser oder einem organischen Lösungsmittel, bevorzugt Aceton, Acetonitril, Benzen, Dioxan, Diethylenglykoldimethylether (Diglyme), Tetrahydrofuran oder Gemischen davon durchgeführt wird.

16. Verfahren nach Anspruch 15, wobei der pH im Bereich von 2 bis 12, bevorzugt zwischen 8,5 und 10 liegt, wenn Wasser ein Teil des Lösungsmittels ist.

17. Verfahren nach einem oder mehreren der Ansprüche 1 bis 16, wobei die Temperatur der Reaktion im Bereich von -30 °C bis 100 °C, bevorzugt zwischen -10 °C und 50 °C liegt.

18. Verfahren nach einem oder mehreren der vorangehenden Ansprüche, wobei der Katalysator danach zurückgewonnen und für anschließende Oxidationen wieder verwendet wird.

## Revendications

1. Procédé d'oxydation d'alcools par des dérivés de formule générale (I) 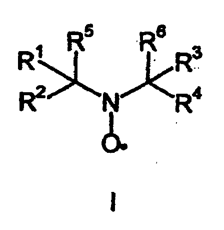 dans laquelle
R¹, R², R³, R⁴, R⁵, R⁶ sont indépendamment l'un de l'autre un (C₁-C₈)-alkyle, un (C₁-C₈)-alkoxy, un (C₆-C₁₈)-aryle, un (C₇-C₁₉)-aralkyl, un (C₆-C₁₈)-aryl-(C₁-C₈)-alkyle, un (C₃-C₁₈)-hétéroaryle, un (C₄-C₁₉)-hétéroaralkyle, un (C₃-C₁₈)-hétéroaryle-(C₁-C₈)-allyle, un (C₃-C₈)-cycloalkyle, un (C₃-C₈)-cycloalkyle-(C₁-C₈)-alkyle, un (C₁-C₈)-alkyle-(C₃-C₈)-cycloalkyle, ou bien R⁵ et R⁶ sont reliés ensemble par l'intermédiaire d'une chaîne (C₁-C₃)-alkyle qui peut être substituée par un ou plusieurs R¹, N[(C₁-C₈)-alkyle]₂, (C₁-C₈)-amido, (C₁-C₈)-alkyloxy carboxyle, (C₆-C₁₈)-aryloxy carboxyle, nitrile, Hal, =0, ou partie d'un polymère, en présence d'un catalyseur métallique sur support et un co-oxydant.

2. Procédé selon la revendication 1,
dans lequel
R¹ à R⁴ sont des (C₁-C₈)-alkyle et R⁵ et R⁶ sont reliés par l'intermédiaire d'une chaîne (C₁-C₃)-alkyle qui peut être substituée par un ou plusieurs groupes R¹, N[(C₁-C₈)-alkyl]₂, (C₁-C₈)-amido, (C₁-C₈)-alkyloxy carboxyle, (C₆-C₁₈)-aryloxy carboxyle, nitrile, Hal, =0, partie d'un polymère.

3. Procédé selon l'une quelconque des revendications 1 ou 2,
dans lequel
R¹ à R⁴ sont un méthyle, R⁵ et R⁶ sont reliés par l'intermédiaire d'une chaîne alkyle en C₃.

4. Procédé selon l'une quelconque des revendications 1 à 3,
dans lequel
le dérivé de formule générale I est employé dans la plage de 0,01 % molaire à 10 % molaire, de préférence entre 0,1 % molaire et 4 % molaire basé sur le substrat.

5. Procédé selon l'une quelconque des revendications 1 à 4,
dans lequel
l'alcool utilisé inclut une fonction alcool primaire.

6. Procédé selon la revendication 5,
dans lequel
l'alcool est oxydé en l'acide carboxylique correspondant.

7. Procédé selon la revendication 1,
dans lequel
le catalyseur métallique sur support inclut des métaux à un degré d'oxydation ± 0 ou des oxydes métalliques.

8. Procédé selon la revendication 7,
dans lequel
les métaux utilisés sont l'argent, le chrome, le palladium, le bismuth, le fer, le vanadium, le cuivre, le cobalt, le nickel, le molybdène, le manganèse, le ruthénium ou l'osmium ou des alliages de ceux-ci.

9. Procédé selon la revendication 7,
dans lequel
les oxydes métalliques utilisés sont MOₓ avec M=argent, chrome, palladium, bismuth, fer, vanadium, cuivre, cobalt, nickel, molybdène, manganèse, ruthénium ou osmium ou des mélanges de ceux-ci ou des oxydes métalliques mixtes de ceux-ci.

10. Procédé selon l'une quelconque des revendications 1 à 9,
dans lequel
le support utilisé consiste en des matériaux micro- ou mesoporeux.

11. Procédé selon la revendication 10,
dans lequel
le matériau microporeux peut être dérivé d'un matériau zéolithique et le matériau mesoporeux peut être de la silice, de l'aluminium, une silice-alumine amorphe, un alumino phosphate, de l'oxyde de titane ou de la celite ou du carbone.

12. Procédé selon l'une quelconque des revendications 1 à 11,
dans lequel
le rapport du métal au support se situe dans la gamme de 0,05 % en poids à 50 % en poids.

13. Procédé selon l'une quelconque des revendications 1 à 12,
dans lequel
le co-oxydant est choisi dans le groupe qui consiste en le peroxyde d'hydrogène, l'oxone, l'ozone, les perborates, les percarbonates, l'oxygène, le 4-oxyde de N-méthyl morpholine, les peroxodisulfates ou caroates.

14. Procédé selon l'une quelconque des revendications 1 à 13,
dans lequel
le rapport entre le co-oxydant et le substrat se situe dans la plage de 1 à 20, de préférence entre 1 et 10 équivalents.

15. Procédé selon l'une quelconque des revendications 1 à 14,
dans lequel
la réaction est effectuée dans l'eau ou dans un solvant organique, de préférence l'acétone, l'acétonitrile, le benzène, le dioxane, le diglyme, le tétrahydrofurane ou des mélanges de ceux-ci.

16. Procédé selon la revendication 15,
dans lequel
si l'eau est une partie du solvant, le pH se situe dans la plage de 2 à 12, de préférence entre 8,5 et 10.

17. Procédé selon l'une quelconque des revendications 1 à 16,
dans lequel
la température de la réaction se situe dans la plage allant de -30°C à 100°C, de préférence entre -10°C et 50°C.

18. Procédé selon l'une quelconque des revendications précédentes,
dans lequel
le catalyseur est récupéré après la réaction et est réutilisé pour des oxydations ultérieures.
